# EUROPÄISCHE PATENTANMELDUNG

(11) **EP 1 479 450 A2**
(43) Veröffentlichungstag der Anmeldung: **24.11.2004**
(21) Anmeldenummer: 04010041.4
(22) Anmeldetag: 28.04.2004
(51) Int. Cl.: B05B 11/02, A61M 15/00, A61M 15/08

(54) **Dosiervorrichtung mit einer Einhub-Pumpeinrichtung**

(30) Priorität: 20.05.2003 DE 10323603
(71) Anmelder: Ing. Erich Pfeiffer GmbH, 78315 Radolfzell (DE)
(72) Erfinder: Stadelhofer, Peter, 78224 Singen (DE)
(74) Vertreter: Patentanwälte Ruff, Wilhelm, Beier, Dauster & Partner

(57) **Zusammenfassung**

Eine Dosiervorrichtung mit einer Pumpeinrichtung, die wenigstens ein Medium in einen längs einer Pumpachse verlaufenden Pumpkanal fördert, sowie mit einem wenigstens eine Applikationsöffnung aufweisenden Applikator, ist bekannt.

Erfindungsgemäß weist der Applikator wenigstens einen Strömungskanal auf, der den Pumpkanal mit der Applikationsöffnung verbindet und der quer zu der Pumpachse ausgerichtet ist, und dass die Applikationsöffnung seitlich an dem Applikator angeordnet ist.

Einsatz für pharmazeutische Anwendungszwecke.

## Beschreibung

Die Erfindung betrifft eine Dosiervorrichtung mit einer Pumpeinrichtung, die wenigstens ein Medium in einen längs einer Pumpachse verlaufenden Pumpkanal fördert, sowie mit einem wenigstens eine Applikationsöffnung aufweisenden Applikator.

Eine solche Dosiervorrichtung ist aus der DE 197 00 437 A1 bekannt. Die bekannte Dosiervorrichtung weist einen Medienspeicher auf, der mittels eines Kolbenstopfens verschlossen ist. Ein Applikator ist relativ zu dem Medienspeicher und relativ zu dem Kolbenstopfen schubbeweglich angeordnet. Der Applikator ist als Nasenadapter ausgeführt und weist eine Fingerauflage auf, um eine Betätigung der Dosiervorrichtung zu ermöglichen. In dem Applikator ist ein Pumpkanal vorgesehen, der zum Medienspeicher hin in einer Hohlnadel endet. Mittels der Hohlnadel ist der Kolbenstopfen durchstechbar. Durch die Relativbewegung zwischen dem Applikator und dem Medienspeicher wird nach dem Durchstechen des Kolbenstopfens der Kolbenstopfen in Richtung des Mediums gedrückt, wodurch dieses komprimiert und durch den Pumpkanal zu einer Applikationsöffnung des Nasenadapters gefördert wird. Die Applikationsöffnung ist koaxial zur Pumpachse an einer Außenseite des Nasenadapters vorgesehen.

Aufgabe der Erfindung ist es, eine Dosiervorrichtung der eingangs genannten Art zu schaffen, die für eine sublinguale Anwendung besonders geeignet ist.

Diese Aufgabe wird dadurch gelöst, dass der Applikator wenigstens einen Strömungskanal aufweist, der den Pumpkanal mit der Applikationsöffnung verbindet und der quer zu der Pumpachse ausgerichtet ist, und dass die Applikationsöffnung seitlich an dem Applikator angeordnet ist. Durch die erfindungsgemäße Lösung erfolgt eine Umlenkung des Mediumstromes von der Pumpachse ausgehend zur Seite hin und damit zumindest weitgehend radial. Die erfindungsgemäße Lösung eignet sich insbesondere für eine Ausführung, bei der die Pumpeinrichtung als Schubkolbenpumpe gestaltet ist und manuell betätigt wird. Die Pumpfunktion entspricht der aus der DE 197 00 437 A1 bekannten Dosierpumpe. Für eine orale und insbesondere sublinguale Anwendung kann die Dosiervorrichtung in einfacher Weise aufrecht stehend vor einem Mund einer zu versorgenden Person platziert werden. Durch die seitlich angeordnete Applikationsöffnung wird bei einer Pumpbetätigung in etwa aufrechtem Zustand der Dosiervorrichtung zwangsläufig eine Applikation in den Mund hinein, vorzugsweise unter eine Zunge, erzielt. Vorzugsweise wird ein flüssiges Medium appliziert. Dies kann bevorzugt durch Zerstäubung oder auch durch Tropfung eingebracht werden. In besonders bevorzugter Ausführung ist eine Applikationsachse der Applikationsöffnung für das Medium etwa parallel zu Auflageachsen der auf einer Fingerauflage des Applikators aufliegenden Fingerabschnitte der Bedienperson ausgerichtet. Alternativ ist es möglich, die Applikationsachse rechtwinklig zu den Auflageachsen zweier paralleler Finger, vorzugsweise eines Zeigefingers und eines Mittelfingers, auszurichten, wobei bei dieser Ausführungsform selbstverständlich dennoch eine Anordnung der Applikationsöffnung seitlich und damit im wesentlichen radial zu der Applikatorachse, d.h. zu der Pumpachse, vorgesehen ist. Die erfindungsgemäße Lösung eignet sich insbesondere für pharmazeutische Anwendungszwecke, aber auch für kosmetische oder andere Einsatzgebiete.

In Ausgestaltung der Erfindung ist die wenigstens eine Applikationsöffnung in einer Sprühdüse vorgesehen, die ausgangsseitig des Strömungskanales in dem Applikator angeordnet ist. Dadurch ist es möglich, eine Zerstäubung des wenigstens einen Mediums zu erzielen. Je nach Ausbildung der Sprühdüse ist eine unterschiedliche Sprühcharakteristik erzielbar. Es ist auch möglich, die Sprühdüse verstellbar zu gestalten, um variable Sprühcharakteristika erreichen zu können.

In weiterer Ausgestaltung der Erfindung ist die Sprühdüse einstückig in dem Applikator integriert. Vorzugsweise stellt der Applikator ein Gehäuseteil aus Kunststoff dar, in dem die Sprühdüse einstückig ausgeformt ist.

In weiterer Ausgestaltung der Erfindung ist die Sprühdüse als separates Bauteil hergestellt und mit dem Applikator verbunden. Bei dieser Ausführungsform ist in dem Applikator, der vorzugsweise als Kunststoffbauteil gestaltet ist, ein Aufnahmebereich geformt, der ein Ein- oder Ansetzen wie auch ein Fixieren der Sprühdüse ermöglicht. Hierzu sind an der Sprühdüse und/oder an oder in dem Aufnahmebereich des Applikators vorzugsweise entsprechende Führungs-, Sicherungs- oder Rastbereiche einstückig angeformt, so dass für das Einsetzen, die Justierung und die Fixierung der Sprühdüse keine zusätzlichen Bauteile benötigt werden.

In weiterer Ausgestaltung der Erfindung ist dem Strömungskanal wenigstens ein Strömungsleitmittel, insbesondere ein Füllstück, zugeordnet. Durch das Strömungsleitmittel ist es möglich, definierte Applikationen zu erzeugen. So ist es insbesondere durch ein Füllstück möglich, einen Ringraum unmittelbar vor einem Düsenaustritt zu schaffen, der in Verbindung mit einer geeigneten Düsengeometrie eine gewünschte Sprühcharakteristik erzeugt.

In weiterer Ausgestaltung der Erfindung ist das Strömungsleitmittel einstückig in dem Applikator integriert. Diese Ausgestaltung ist insbesondere vorteilhaft, falls der Applikator als Kunststoffbauteil gestaltet ist und das wenigstens eine Strömungsleitmittel einstückiger Teil oder Abschnitt dieses Kunststoffbauteiles ist. In vorteilhafter Weise ist ein Füllstück vorgesehen, das einstückig in dem Applikator integriert ist.

In weiterer Ausgestaltung der Erfindung ist das Strömungsleitmittel als separates Formteil hergestellt und in den Applikator eingesetzt. Vorzugsweise wird das Strömungsleitmittel dem Strömungskanal zugeordnet. Bei einer bevorzugten Ausführungsform ist als Strömungsleitmittel ein zumindest weitgehend zylindrisches Füllstück vorgesehen, das koaxial zu einer Längsachse des Strömungskanales von einer Seite her in den Applikator eingesetzt wird.

In weiterer Ausgestaltung der Erfindung entspricht der Querschnitt der Applikationsöffnung dem Querschnitt des Strömungskanales oder ist gegenüber diesem lediglich geringfügig verändert. Ein Übergang des Strömungskanales zu der Applikationsöffnung verläuft somit im wesentlichen ohne Änderung des Strömungsquerschnittes und damit insbesondere zumindest weitgehend ohne zusätzliche Strömungsleitfunktion. Durch diese Ausgestaltung ist insbesondere eine Applikation durch Tropfung zuverlässig möglich.

In weiterer Ausgestaltung der Erfindung ist dem Applikator eine Fingerauflage zugeordnet, die derart relativ zu einer Applikationsachse der Applikationsöffnung angeordnet ist, dass bei einer Anlage wenigstens eines auf der Fingerauflage aufliegenden Fingerabschnittes eines Bedieners unterhalb einer Unterlippe des Bedieners eine Mediumapplikation in den Mund des Bedieners erfolgen kann. Dies ist eine ergonomisch besonders günstige Ausführung. Dabei legt der Bediener insbesondere vordere Gelenkabschnitte seines Zeige- und seines Mittelfingers, die auf der Fingerauflage aufliegen und vorzugsweise den Applikator flankieren, unterhalb der Unterlippe und damit knapp oberhalb seines Kinns an das Gesicht an. Vorteilhaft ist die Applikationsachse, d.h. eine Hauptaustrittsachse der Applikationsöffnung zum Ausbringen des Mediums, etwa auf Mundhöhe positioniert, so dass bei geöffnetem Mund zwangsläufig eine Applikation des Mediums in den Mund hinein erfolgt. Alternativ kann der Bediener die Dosiervorrichtung um 90° verdreht unterhalb der Unterlippe ansetzen. Dann ist vorzugsweise lediglich ein Zeigefinger zusammen mit dem entsprechenden Teil der Fingerauflage zwischen Kinn und Mund an das Gesicht angelegt, während der den Applikator auf der gegenüberliegenden Seite flankierende und auf der Fingerauflage aufliegende Mittelfinger zum Gesicht beabstandet ist. Bei dieser Variante ist die Applikationsachse zwar ebenfalls radial zu der Pumpachse, aber rechtwinklig zu den Fingerauflageachsen der auf der Fingerauflage aufliegenden Finger ausgerichtet. Selbstverständlich ist die rechtwinklige Ausrichtung so gewählt, dass die Applikationsachse zum Mund hin und nicht vom Mund weg zeigt. Dies bedeutet, dass die Applikationsachse von der Pumpachse ausgehend in der Richtung radial abragt, in der die seitliche Auflagefläche für den Zeigefinger auf der Fingerauflage gebildet ist. Diese beiden Varianten sind alternativ realisiert, wenn die Applikationsachse parallel oder rechtwinklig zu der Fingerauflageachse wenigstens eines Fingerabschnittes verläuft.

In weiterer Ausgestaltung der Erfindung ist die Applikationsachse in einem - auf die Pumpachse bezogen - axialen Abstand zu der Fingerauflage ausgerichtet, der so gewählt ist, dass bei einer Positionierung der Dosiervorrichtung mit unterhalb der Unterlippe des Bedieners angeordneter Fingerauflage die Applikationsöffnung in Richtung und auf Höhe eines geöffneten Mundes des Bedieners ausgerichtet ist. Diese Ausgestaltung gewährleistet, dass der axiale Abstand der Applikationsachse zur Fingerauflage weder zu groß noch zu klein ist. Dadurch ist sichergestellt, dass bei richtiger Fingeranlage unterhalb der Unterlippe tatsächlich auch eine Applikation in den Mund hinein erfolgt.

Gemäß einer bevorzugten Ausführungsform ist vorgesehen, dass der Applikator einen Kolbenfortsatz aufweist, der in einer unbelasteten Ruhestellung des Applikators radial geführt zumindest teilweise axial in einen offenen Zylinderabschnitt eines Mediumspeichers hineinragt. Durch das radial geführte, teilweise axiale Hineinragen eines Kolbenfortsatzes des Applikators in einen offenen Zylinderabschnitt eines Mediumspeichers in einer unbelasteten Ruhestellung des Applikators ist gewährleistet, dass unabhängig von einer Intensität einer entsprechenden Betätigungsbewegung ein sicheres und zentriertes Durchstechen des Kolbenstopfens erreicht wird. Undichtigkeiten und mangelhafte Dosierungen des Dosiersystems werden dadurch zuverlässig vermieden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in einer Schnittdarstellung eine erste Ausführungsform einer erfindungsgemäßen Dosiervorrichtung für eine Mehrfachanwendung,
- Fig. 2: eine relativ zu einer Mittellängsachse der Dosiervorrichtung nach Fig. 1 um 90° verdrehte Schnittdarstellung der Dosiervorrichtung nach Fig. 1,
- Fig. 3: in einer Schnittdarstellung eine weitere Ausführungsform einer Dosiervorrichtung ähnlich Fig. 1,
- Fig. 4: eine um 90° verdrehte Schnittdarstellung der Dosiervorrichtung nach Fig. 3,
- Fig. 5: in einer Schnittdarstellung eine weitere Ausführungsform einer Dosiervorrichtung ähnlich Fig. 3 und 4,
- Fig. 6: die Dosiervorrichtung nach Fig. 5 in einer um 90° verdrehten Schnittdarstellung,
- Fig. 7: eine weitere Ausführungsform einer erfindungsgemäßen Dosiervorrichtung in einer Schnittdarstellung,
- Fig. 8: die Dosiervorrichtung nach Fig. 7 in einer um 90° verdrehten Schnittdarstellung,
- Fig. 9: eine weitere Ausführungsform einer erfindungsgemäßen Dosiervorrichtung mit Schutzkappe in geschnittener Darstellung,
- Fig. 10: die Dosiervorrichtung nach Fig. 9 in einer um 90° versetzten Schnittansicht und
- Fig. 11: in gegenüber den stark vergrößerten Darstellungen nach den Fig. 1 bis 10 verkleinerte Darstellung einer weiteren Ausführungsform einer erfindungsgemäßen Dosiervorrichtung, die für eine Tropfapplikation ausgeführt ist.

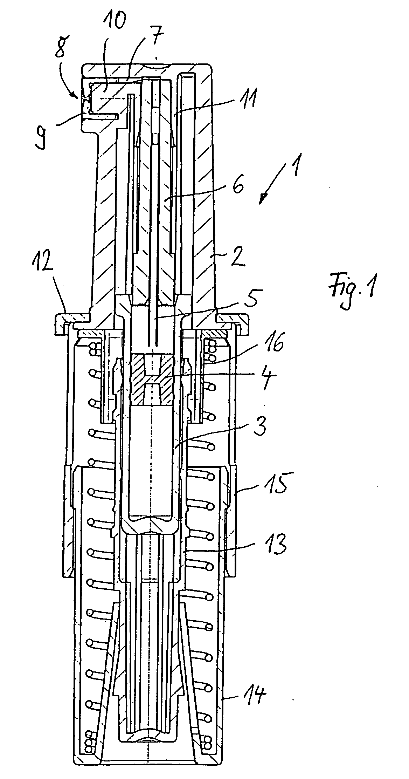

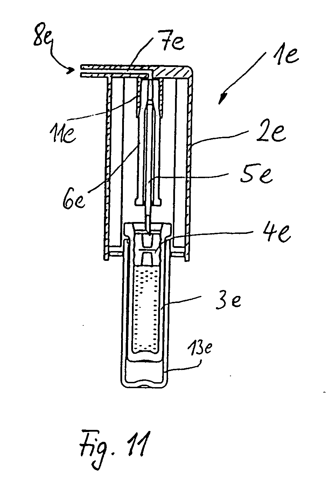

Die nachfolgend anhand der Fig. 1 bis 11 beschriebenen unterschiedlichen Ausführungsformen von Dosiervorrichtungen sind vorzugsweise für pharmazeutische Anwendungsbereiche vorgesehen und dienen dazu, entsprechende Wirkstoffe mittels eines flüssigen Mediums zu applizieren. Bei den dargestellten Ausführungsbeispielen sind die Wirkstoffe in dem flüssigen Medium gelöst. Die erfindungsgemäße Lösung wie auch die nachfolgend beschriebenen Ausführungsbeispiele eignen sich mit geringfügigen Abwandlungen auch dazu, die Wirkstoffe als Festkörper, d.h. insbesondere in Pulverform, vorzusehen und diese vor einem entsprechenden Applikationsvorgang mit einem flüssigen Trägermedium in Verbindung zu bringen und so eine Lösung der pulverförmigen Wirkstoffe in dem Medium zu erzielen. Die anschließende Applikation entspricht den nachfolgend beschriebenen Ausführungsbeispielen. Die anhand der Fig. 1 bis 11 dargestellten Ausführungsbeispiele sind als Einfachdosierer oder als Zweifachdosierer ausgeführt. Dies bedeutet, dass das bevorratete, flüssige Medium in einem einzigen Dosiervorgang bzw. in zwei voneinander getrennten Dosiervorgängen ausgebracht werden kann. Die Einfachdosierer eignen sich somit für eine einfache Anwendung, die Zweifachdosierer für eine doppelte Anwendung. Alle Dosiervorrichtungen gemäß den Fig. 1 bis 11 sind für orale Applikationen, insbesondere für sublinguale Anwendungen vorgesehen.

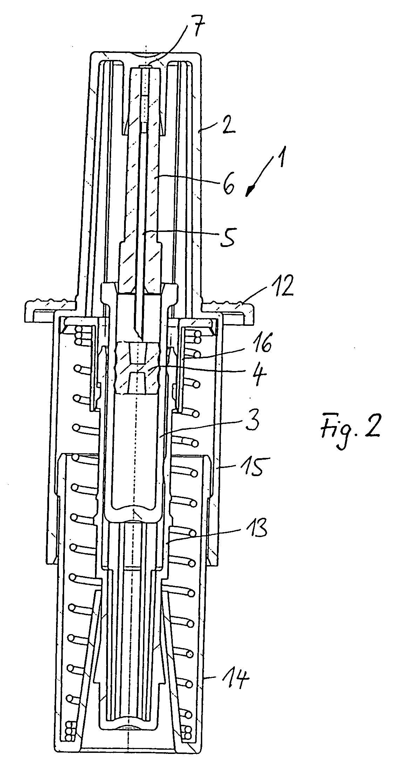

Alle Dosiervorrichtungen gemäß den Fig. 1 bis 11 sind zumindest weitgehend aus Kunststoffteilen aufgebaut, die im Spritzgussverfahren hergestellt sind. Lediglich nachfolgend näher beschriebene Hohlnadeln 5 bis 5e sind bei allen Ausführungsformen in grundsätzlich bekannter Weise aus Metall hergestellt. Der Mediumspeicher 3 bis 3e ist vorzugsweise aus Glas hergestellt. Bei dem Ausführungsbeispiel nach den Fig. 1 und 2 ist zudem eine als Federspeicher bzw. als Rückstellfeder dienende Schraubendruckfeder ebenfalls aus Metall, vorzugsweise aus einem Federstahl, hergestellt. Die Dosiervorrichtungen nach den Fig. 1 bis 10 sind in vergrößertem Maßstab dargestellt, um die einzelnen Bauteile der Dosiervorrichtungen deutlich und detailliert erkennen zu können.

Die Dosiervorrichtung nach den Fig. 1 und 2 weist eine Pumpeinrichtung auf, die als Schubkolbenpumpe gestaltet ist. Die Dosiervorrichtung 1 weist einen hohlzylindrisch gestalteten Mediumspeicher 3 auf, der zu einer in den Fig. 1 und 2 nach oben gerichteten Oberseite hin offen ist. In dem Medienspeicher 3 ist ein Kolbenstopfen 4 dicht, aber verschiebbar positioniert, der aus einem elastischen Material, insbesondere aus einem Elastomer, hergestellt ist. In dem durch den Mediumspeicher 3 und den Kolbenstopfen 4 begrenzten Raum ist ein mit wenigstens einem pharmazeutischen Wirkstoff versehenes flüssiges Medium vorgesehen. Der Mediumspeicher 3 ist in einem Betätigungszylinder 13 aufgenommen und bodenseitig abgestützt. Der Betätigungszylinder 13 ist von einem hülsenartigen Betätigungsglied 14 umgeben, das längs einer den Mediumspeicher 3 koaxial durchsetzenden Pumpachse in einem Aufnahmekranz 15 koaxial zur Pumpachse schubbeweglich gelagert ist. Der Betätigungszylinder 13 ist in einer Stützhülse 16 gehalten und verschiebbar gelagert. Für die entsprechenden Halte- oder Fixier- oder Anschlagfunktionen sind an den verschiedenen Bauteilen Rastnasen, Anschlagschultern und andere Profilabschnitte vorgesehen, um bei einer Betätigung der Dosiervorrichtung 1 ein zeitlich versetztes Zusammenwirken der Bauteile zu erzielen. Die Dosiervorrichtung 1 nach den Fig. 1 und 2 ist für eine Zweifachdosierung ausgelegt.

Der Aufnahmekranz 15 ist Teil eines Applikators 2. Auf dem Applikator 2 ist eine Fingerauflage 12 als getrenntes Bauteil lösbar fixiert. Der Applikator 2 ist hülsen- oder glockenartig gestaltet und weist in seinem Inneren nicht näher bezeichnete, achsparallel zur Pumpachse verlaufende Führungsnuten auf, in denen ein oberer Randbereich des Mediumspeichers 3 positioniert ist, um eine axiale Verschiebbarkeit des Applikators 2 relativ zu dem Mediumspeicher 3 zu gewährleisten. Der Applikator 2 weist in seinem Inneren im Bereich seiner Oberseite eine einstückig angeformte Aufnahmehülse 11 auf, die koaxial zur Pumpachse nach unten abragt. In die Aufnahmehülse 11 ist ein im wesentlichen zylindrisches Kolbenteil 6 axial eingesetzt, in dem ein koaxial zur Pumpachse verlaufender Pumpkanal ausgebildet ist. In den Pumpkanal ist die Hohlnadel 5 koaxial eingesetzt, wobei eine Spitze der Hohlnadel 5 nach unten aus dem Kolbenteil 6 herausragt. Die Spitze der Hohlnadel 5 dient dazu, bei einer Betätigung der Dosiervorrichtung 1 den Kolbenstopfen 4 zu durchstechen. In dem oberen Stirnbereich geht der Pumpkanal in einen rechtwinklig und damit radial zur Pumpachse ausgerichteten Strömungskanal 7 über, der in einer mit einem als Austrittsöffnung dienenden Düsenaustritt 8 versehenen Sprühdüse 9 mündet. Der Strömungskanal 7 ist einstückig in dem Applikator 2 ausgebildet und setzt sich zum Düsenaustritt 8 hin in einen zur Seite des Applikators 2 hin offenen Ringraum fort. Der Ringraum wird durch ein einstückig im Applikator 2 ausgeformtes, zylindrisches Füllstück 10 gebildet, das radial zur Pumpachse nach außen gerichtet ist. Der zur Seite des Applikators 2 offene Ringraum wird durch einen Düseneinsatz verschlossen, der die Sprühdüse 9 bildet. In dem Düseneinsatz ist der Düsenaustritt 8 einstückig angeformt. Der Düseneinsatz ist aus Kunststoff hergestellt. Der Düseneinsatz wird in einfacher Weise radial zur Pumpachse von außen her in den Ringraum eingesetzt und dort durch geeignete Mittel lösbar oder unlösbar fixiert. Die Fixierung kann kraftschlüssig, stoffschlüssig oder formschlüssig erfolgen. Eine Hauptsprührichtung der Sprühdüse 9 wird durch die Symmetrieachse des Füllstückes 10 erzielt und ist somit quer zur Pumpachse, d.h. radial zur Pumpachse, ausgerichtet. In Umfangsrichtung gesehen ist die Lage der Sprühachse so positioniert, dass die Sprühachse etwa parallel zu imaginären Auflageachsen für auf der Fingerauflage 12 aufliegende Finger positioniert ist. Falls gemäß Fig. 2 ein Zeigefinger und ein Mittelfinger beidseitig des Applikators 2 auf der Fingerauflage 12 aufliegen, so sind die beiden Finger etwa parallel zueinander ausgerichtet und flankieren den Applikator 2 von beiden Seiten. Etwa parallel zur Ausrichtung der Finger ist auch die Sprühdüse 9 ausgerichtet, so dass die Sprühachse etwa parallel zu der Ausrichtung der auf der Fingerauflage 12 aufliegenden Fingerabschnitte verläuft. Dabei wird die Dosiervorrichtung 1 vorzugsweise so ergriffen, dass der wird die Dosiervorrichtung 1 vorzugsweise so ergriffen, dass der Strömungskanal und damit der Ringraum des Applikators 2 sich in Richtung der Fingerkuppen öffnet. Für einen Anwender ist es nun möglich, die Fingerspitzen, d.h. die Fingerkuppen, der auf der Fingerauflage 12 aufliegenden Zeige- und Mittelfinger unterhalb seiner Unterlippe anzulegen, wodurch die Sprühdüse 9 zwangsläufig in Richtung des offenen Mundes ragt. Dadurch ist eine ergonomisch günstige orale und insbesondere sublinguale Applikation ermöglicht.

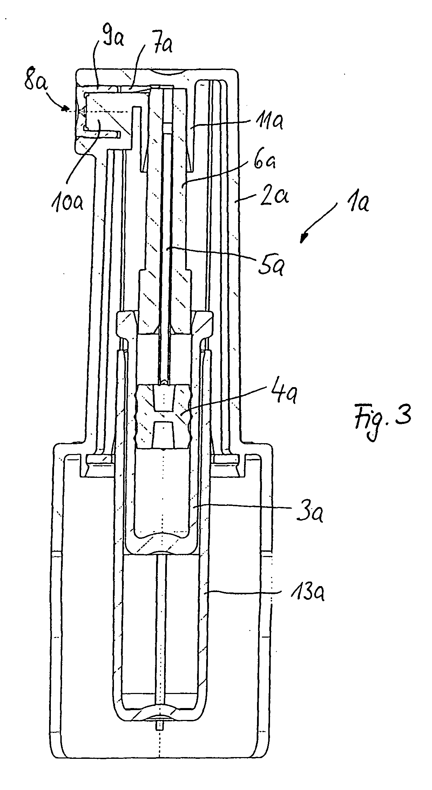

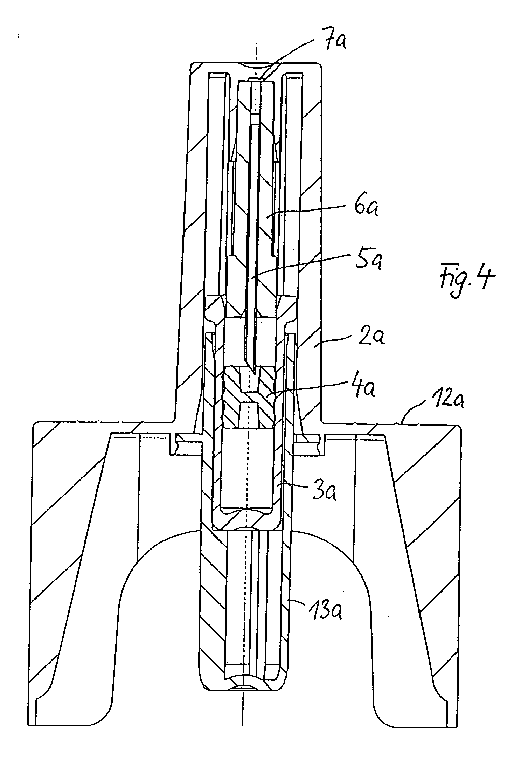

Das Funktionsprinzip der Dosiervorrichtung 1a nach den Fig. 3 und 4 entspricht im wesentlichen der anhand der Fig. 1 und 2 beschriebenen Dosiervorrichtung 1. Auch hier ist als Pumpeinrichtung eine Schubkolbenpumpe vorgesehen. Wesentlicher Unterschied bei der Dosiervorrichtung 1a ist es zum einen, dass diese nur für eine Einmalanwendung vorgesehen ist. Weiterer Unterschied ist es, dass an dem Applikator 2a auch die Fingerauflage 12a einstückig angeformt ist. Das den Mediumspeicher 3 aufnehmende Betätigungsglied 13a ist so ausgeführt, dass es direkt von unten her mit einem Daumen des Bedieners nach oben gedrückt werden kann. Das Betätigungsglied 13a ist in grundsätzlich bekannter Weise durch Raststege gehalten, die bei einer Druckbetätigung durch einen Daumen abbrechen und eine entsprechende Schubbewegung ermöglichen. Auch bei der Ausführungsform nach den Fig. 3 und 4 ist eine Sprühachse des Düsenaustrittes 8a von der Sprühdüse 9a derart quer und radial zur Pumpachse ausgerichtet, dass sie parallel zu den imaginären Auflageachsen der entsprechenden Fingerabschnitte von Zeigefinger und Mittelfinger verläuft. Dadurch ergeben sich die gleichen Vorteile und Applikationsfunktionen wie bei dem Ausführungsbeispiel nach den Fig. 1 und 2. Bau- oder funktionsgleiche Teile sind mit den gleichen Bezugszeichen wie beim Ausführungsbeispiel nach den Fig. 1 und 2, jedoch unter Hinzufügung des Buchstabens a, versehen. Anhand der Fig. 3 ist gut erkennbar, dass der hülsenartige Düseneinsatz der Sprühdüse 9a im eingesetzten Zustand formschlüssig in dem Applikator 2a gehalten ist, da ein vorderer Randbereich des in dem Applikator 2a ausgebildeten Ringraumes mit in den Ringraum geringfügig hineinragenden Hinterschneidungen versehen ist. Zudem ist die Gestaltung des Düseneinsatzes 9a erkennbar. Zwischen Füllstück 10a und Düseneinsatz 9a ergibt sich in einem vorderen Stirnbereich des Füllstückes 10a ein umlaufender Ringspalt, der zu dem zentral ausgerichteten Düsenaustritt 8a offen ist. Der Strömungskanal 7a steht mit dem Ringspalt in nicht näher dargestellter Weise über entsprechende Strömungskanalabschnitte in Verbindung, die aus den Fig. 3 und 4 nicht erkennbar sind. Der Strömungskanal 7a zweigt von dem Pumpkanal oberhalb des Füllstückes 10a ab, so dass das flüssige Medium in einem oberen Bereich des Ringraumes an diesen herangeführt wird.

Sowohl bei der Ausführungsform nach den Fig. 1 und 2 als auch bei der Ausführungsform nach den Fig. 3 und 4 ist der Kolbenteil 6, 6a von seiner auf eine Pumpenachse bezogenen axialen Länge her so gestaltet, dass er in einer unbelasteten Ruheposition des Applikators, wie sie in den Fig. 1 bis 4 dargestellt ist, zumindest abschnittsweise axial in die offene Stirnseite des Mediumspeichers 3, 3a hineinragt. Vorzugsweise ist außerdem der hineinragende Abschnitt des Kolbenteiles 6, 6a in seinem Querschnitt so gewählt, dass er in dieser Ruheposition weitgehend spielfrei zentriert zu einer Mittellängsachse des Mediumspeichers 3a in diesem ausgerichtet ist (Fig. 3). In einem oberen Stirnrandbereich weist der Mediumspeicher 3, 3a eine sich konisch verjüngende Zentrieröffnung auf, um eine weiter verbesserte Zentrierung des Kolbenteiles 6, 6a bei einer Montage der Dosiervorrichtung vornehmen zu können.

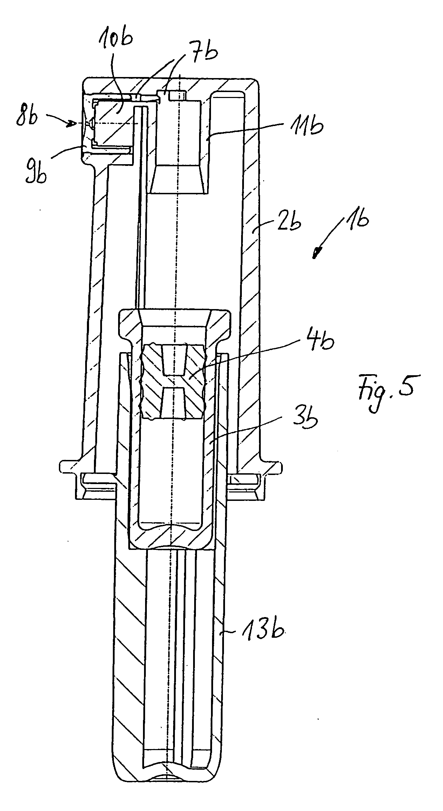

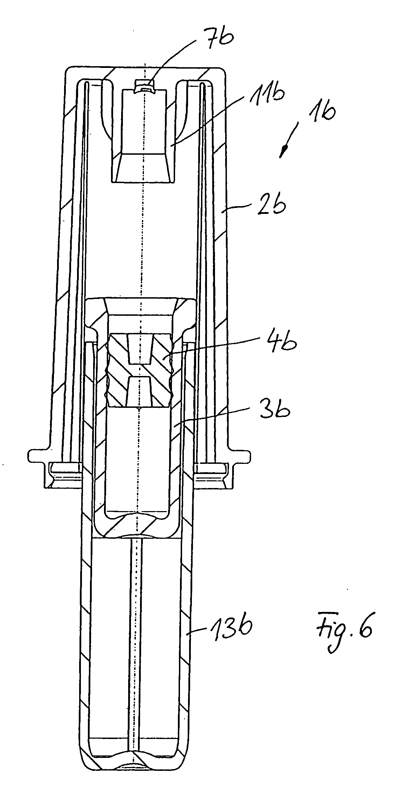

Bei der Ausführungsform nach den Fig. 5 und 6 ist die Dosiervorrichtung 1 b lediglich unvollständig dargestellt. Insbesondere der Kolbenteil und die eingesetzte Hohlnadel sind nicht dargestellt. Von Aufbau und Gestaltung her entspricht der Applikator 2b im wesentlichen den zuvor beschriebenen Applikatoren 2 und 2a. Die Dosiervorrichtung 1b ist als Einwegdosierer ausgeführt, wobei die unvollständig dargestellte Pumpeinrichtung analog der Ausführungsform nach den Fig. 3 und 4 als Schubkolbenpumpe gestaltet ist. Die Aufnahmehülse 11b, in die der Kolbenteil 6 eingesetzt wird, entspricht ebenfalls dem Ausführungsbeispiel nach den Fig. 3 und 4. Gleiches gilt für die Lage des Strömungskanales 7b, für die einstückige Ausformung des Füllstückes 10b, den radial von außen her in einem Ringraum des Strömungskanales 7b und des Applikators 2b fixierten Düseneinsatz der Zerstäuberdüse 9b und die zentrale Lage des Düsenaustrittes 8b. Bezüglich der Offenbarung dieses Ausführungsbeispiels kann daher auf die Offenbarung der Ausführungsformen nach den Fig. 1 bis 4 ergänzend verwiesen werden. Baugleiche oder funktionsgleiche Bauteile sind mit den gleichen Bezugszeichen wie bei den Ausführungsformen nach den Fig. 1 bis 4, jedoch unter Hinzufügung des Buchstabens b versehen.

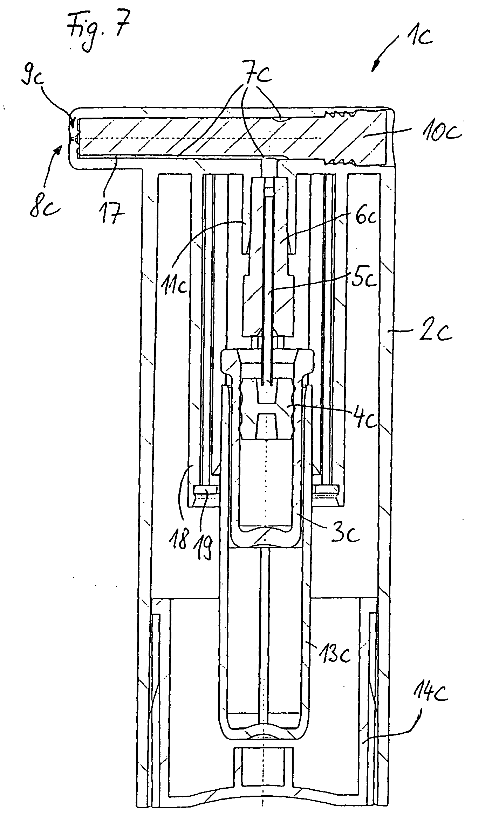

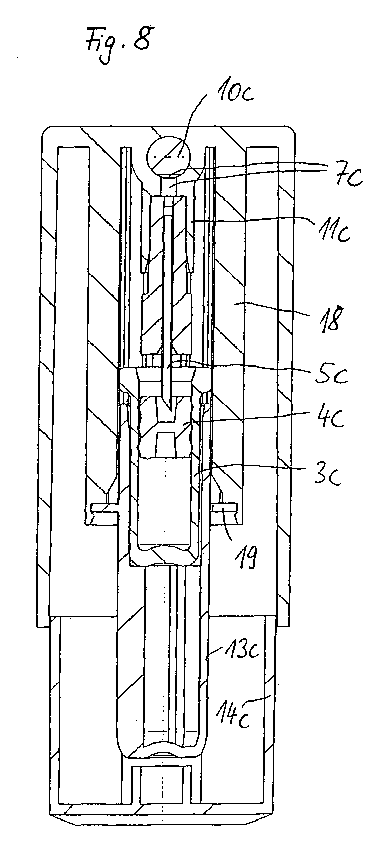

Bei der Ausführungsform nach den Fig. 7 und 8 weist die Dosiervorrichtung 1c eine Pumpeinrichtung auf, die als Schubkolbenpumpe entsprechend den Dosiervorrichtungen 1a und 1b nach den Fig. 3 bis 6 ausgeführt ist. Baugleiche Teile der Schubkolbenpumpe sind mit gleichen Bezugszeichen unter Hinzufügung des Buchstabens c versehen. Wesentlicher Unterschied bei der Ausführungsform nach den Fig. 7 und 8 ist es, dass ein dem Strömungskanal 7c zugeordnetes Füllstück 10c als separates Bauteil von außen her in den Applikator 2c eingesetzt ist. Der Strömungskanal 7c weist eine Strömungsachse auf, die sich quer zu der Pumpachse der Schubkolbenpumpe 3c bis 6c, 13c, 14c erstreckt. Zudem ist ein Düsenaustritt 8c einer nachfolgend näher beschriebenen Zerstäuberdüse einstückig in eine Wandung des Applikators 2c integriert, indem bei der Herstellung des Kunststoffbauteiles des Applikators 2c der Düsenaustritt 8c sowie entsprechende Leitprofilierungen, die als Strömungsleitflächen für eine Zerstäubungsfunktion dienen, mit eingeformt worden sind. Der Strömungskanal 7c ist etwa zylindrisch gestaltet und erstreckt sich in einem oberen Bereich des Applikators 2c radial zur Pumpachse. Der Düsenaustritt 8c ist im Bereich eines quer zur Pumpachse abragenden Nasenfortsatzes ausgebildet, der quer zu einer Wandung des Applikators 2c nach außen abragt. Selbstverständlich ist der Nasenfortsatz ebenfalls einstückiger Bestandteil des Applikators 2c. Der zylindrische Raum, der den Strömungskanal 7c bildet, ist zu dem Düsenaustritt 8c erweitert und zu dieser gegenüberliegenden Seite hin offen. Über die offene Seite ist es möglich, das ebenfalls im wesentlichen zylindrische Füllstück 10c in den zylindrischen Raum einzusetzen, das den Strömungskanal 7c bildet. Das Füllstück 10c weist in seinem dem Düsenaustritt 8c abgewandten Endbereich mehrere umlaufende Dichtlippen im Bereich eines Dichtkopfes auf, um zum einen eine sichere Fixierung des Füllstückes 10c in dem Applikator 2c und zum anderen eine sichere Abdichtung des Strömungskanales 7c zu der dem Düsenaustritt 8c gegenüberliegenden Seite hin zu gewährleisten. Das im wesentlichen zylindrische Füllstück 10c weist einen Durchmesser auf, der zumindest weitgehend dem Durchmesser des Strömungskanales 7c entspricht, um eine spielfreie Positionierung des Füllstückes 10c in dem Strömungskanal 7c zu gewährleisten. Entweder das Füllstück 10c oder der Strömungskanal 7c weisen eine oder mehrere Längsprofilierungen auf, die nut- oder kanalartige Strömungsleitabschnitte 17 definieren. Das Medium wird somit durch die zwischen der Außenkontur des Füllstückes 10c und einer Innenwandung des Strömungskanales 7c verlaufenden und durch die wenigstens eine Längsprofilierung definierten Strömungskanalabschnitte zu ringartigen Strömungsleitabschnitten an einer Innenwandung einer Stirnseite des Nasenfortsatzes geführt, in die der wenigstens eine Strömungsleitabschnitt 17 mündet. Die ringförmigen Strömungsleitabschnitte definieren zudem einen Strömungsweg zu dem zentralen Düsenaustritt 8c. Der Düsenaustritt 8c ist wie auch bei den zuvor beschriebenen Ausführungsbeispielen so gewählt, dass in Verbindung mit den durch ein entsprechendes Füllstück 10 bis 10c und gegebenenfalls zusätzliche Strömungsleitprofilierungen gebildeten Strömungsleitmitteln eine gewünschte Zerstäubungsfunktion erzielt wird. Etwa in axialer Verlängerung des Pumpkanales ist in dem Füllstück 10c eine Ringnut vorgesehen, die den Beginn des Strömungskanalabschnittes 17 - in Strömungsrichtung gesehen- definiert.

Beim Ausführungsbeispiel nach den Fig. 7 und 8 ist im Inneren des Applikators 2c zudem ein Mantelbereich 18 vorgesehen, der die Schubkolbenpumpe koaxial umschließt und in seinem unteren Stirnendbereich eine Rastaufnahme für einen ringartigen Raststeg 19 definiert, der bei einer Druckbelastung auf die Schubkolbenpumpe abreißt und so den Weg für einen Zerstäubungsvorgang frei gibt. Auch die Dosiervorrichtung 1c nach den Fig. 7 und 8 ist für eine Einmaldosierung ausgebildet.

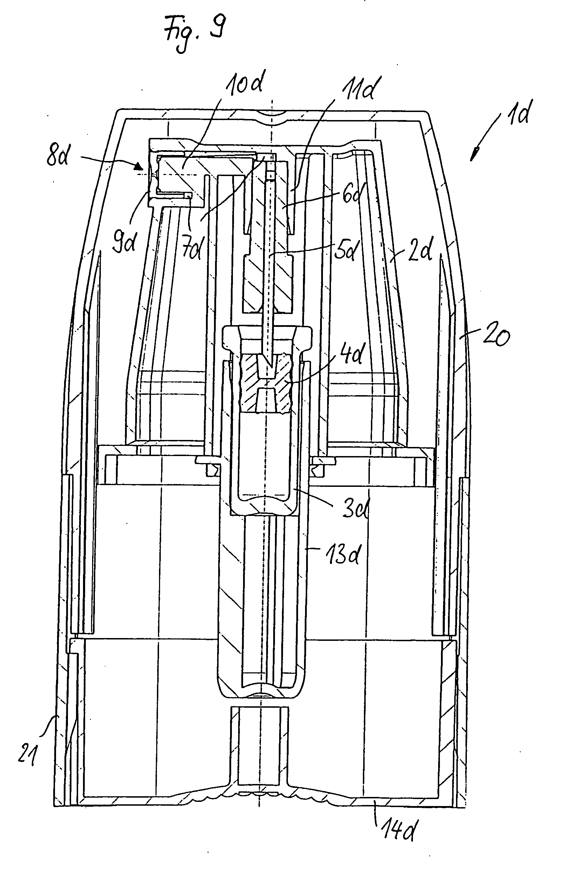

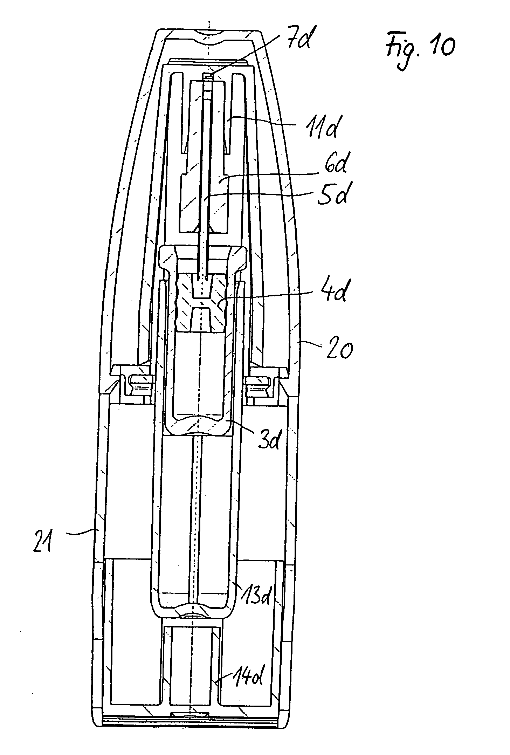

Die Dosiervorrichtung 1d nach den Fig. 9 und 10 entspricht in ihrer Pump- und Zerstäubungsfunktion wie auch in dem Aufbau der zugehörigen Teile der Dosiervorrichtung 1d den zuvor anhand der Fig. 3 bis 6 beschriebenen Ausführungsbeispielen. Baugleiche oder funktionsgleiche Teile sind mit den gleichen Bezugszeichen unter Hinzufügung des Buchstabens d versehen. Bezüglich dieser bau- oder funktionsgleichen Teile wird auf die Offenbarung zu den vorhergehenden Ausführungsformen verwiesen. Wesentlicher Unterschied bei der Dosiervorrichtung 1d ist es, dass die Dosiervorrichtung 1d zusätzlich mit einem Schutzgehäuse 20, 21 versehen ist. Das Schutzgehäuse 20, 21 setzt sich aus einer Schutzkappe 20 und einem Unterteil 21, jeweils aus Kunststoff, zusammen. Die Schutzkappe 20 und das Unterteil 21 sind derart ausgeführt und auf den Applikator 2d abgestimmt, dass sich ohne Abnehmen der Schutzkappe 20 ein Betätigungsschutz ergibt. Dies bedeutet, dass die Dosiervorrichtung 1d nicht betätigt werden kann, so lange die Schutzkappe 20 nicht entfernt ist. Dadurch ergibt sich eine Sicherung der Dosiervorrichtung 1d gegen unbefugte Benutzung, insbesondere eine Kindersicherung. Die Schutzkappe 20 ist mittels nicht näher bezeichneter Rastnasen an entsprechenden Haltestegen des Applikators 2d eingerastet. Durch zumindest geringfügige elastische Verformbarkeit der Schutzkappe 20 ist es möglich, die Verrastung zu lösen und die Schutzkappe 20 zu entfernen. Jetzt ist eine Bedienung der Dosiervorrichtung 1d möglich analog den zuvor beschriebenen Ausführungsformen. Auch die Dosiervorrichtung 1 d ist für eine Einfachdosierung vorgesehen.

Bei der Dosiervorrichtung 1e ist eine Pumpeinrichtung vorgesehen, die als Schubkolbenpumpe entsprechend den zuvor beschriebenen Ausführungsbeispielen ausgeführt ist. Wesentlicher Unterschied ist es, dass bei dieser Ausführungsform der Applikator 2e mit einer Applikationsöffnung 8e versehen ist, die eine Tropffunktion bewirkt. Hierzu ist in einem oberen Stirnbereich des Applikators 2e das den Applikator 2e definierende Gehäuse mittels eines nasenförmigen Seitenfortsatzes zur Seite und damit quer zur Pumpachse hin nach außen fortgesetzt. In diesem oberen Stirnbereich ist ein Strömungskanal 7e eingeformt, der ausgehend von dem zentralen Pumpkanal radial zur Pumpachse bis zur Applikationsöffnung 8e nach außen führt. Der Strömungskanal 7e weist über seine gesamte Länge durchgängig den gleichen Querschnitt auf. Auch die Applikationsöffnung 8e weist einen Querschnitt auf, der dem Querschnitt des Strömungskanales 7e entspricht. Durch diese Ausgestaltung ist es möglich, das Medium aus dem Mediumspeicher 3e tropfenweise zu applizieren. Das tropfenweise Applizieren erfolgt in einfacher Weise durch stetige, manuelle Druckbelastung auf den Betätigungszylinder 13e. Für eine orale Anwendung wird der mit der Applikationsöffnung 8e versehene Seitenfortsatz des Applikators 2e vorzugsweise so relativ zu dem offenen Mund ausgerichtet, dass die austretenden Tropfen in den Mund und vorzugsweise unter die Zunge gelangen.

## Patentansprüche

1. Dosiervorrichtung mit einer Pumpeinrichtung, die wenigstens ein Medium in einen längs einer Pumpachse verlaufenden Pumpkanal fördert, sowie mit einem wenigstens eine Applikationsöffnung aufweisenden Applikator, **dadurch gekennzeichnet, dass** der Applikator (2 bis 2e) wenigstens einen Strömungskanal (7 bis 7e) aufweist, der den Pumpkanal mit der Applikationsöffnung (8 bis 8e) verbindet und der quer zu der Pumpachse ausgerichtet ist, und dass die Applikationsöffnung (8 bis 8e) seitlich an dem Applikator (2 bis 2e) angeordnet ist.

2. Dosiervorrichtung nach Anspruch 1, **dadurch gekennzeichnet, dass** die wenigstens eine Applikationsöffnung (8 bis 8d) in einer Sprühdüse (9 bis 9d) vorgesehen ist, die ausgangsseitig des Strömungskanales (7 bis 7d) in dem Applikator (2 bis 2d) angeordnet ist.

3. Dosiervorrichtung nach Anspruch 2, **dadurch gekennzeichnet, dass** die Sprühdüse (9c) einstückig in dem Applikator (2c) integriert ist.

4. Dosiervorrichtung nach Anspruch 2, **dadurch gekennzeichnet, dass** die Sprühdüse (9 bis 9b, 9d) als separates Bauteil hergestellt und mit dem Applikator (2 bis 2b, 2d) verbunden ist.

5. Dosiervorrichtung nach Anspruch 1, **dadurch gekennzeichnet, dass** dem Strömungskanal wenigstens ein Strömungsleitmittel, insbesondere ein Füllstück (10 bis 10d), zugeordnet ist.

6. Dosiervorrichtung nach Anspruch 5, **dadurch gekennzeichnet, dass** das Strömungsleitmittel (10 bis 10b, 10d) einstückig in dem Applikator (2 bis 2b, 2d) integriert ist.

7. Dosiervorrichtung nach Anspruch 5, **dadurch gekennzeichnet, dass** das Strömungsleitmittel als separates Formteil (10c) hergestellt und in den Applikator (2c) eingesetzt ist.

8. Dosiervorrichtung nach Anspruch 1, **dadurch gekennzeichnet, dass** der Querschnitt der Applikationsöffnung (8e) dem Querschnitt des Strömungskanales (7e) entspricht oder gegenüber diesem lediglich geringfügig verändert ist.

9. Dosiervorrichtung nach Anspruch 1, **dadurch gekennzeichnet, dass** dem Applikator (2, 2a) eine Fingerauflage (12, 12a) zugeordnet ist, die derart relativ zu einer Applikationsachse der Applikationsöffnung (8, 8a) angeordnet ist, dass bei einer Anlage wenigstens eines auf der Fingerauflage (12, 12a) aufliegenden Fingerabschnittes eines Bedieners unterhalb einer Unterlippe des Bedieners eine Mediumapplikation in einen Mund des Bedieners erfolgen kann.

10. Dosiervorrichtung nach Anspruch 9, **dadurch gekennzeichnet, dass** die Applikationsachse parallel oder rechtwinklig zu einer Fingerauflageachse wenigstens eines Fingerabschnittes verläuft.

11. Dosiervorrichtung nach Anspruch 9 oder 10, **dadurch gekennzeichnet, dass** die Applikationsachse in einem - auf die Pumpachse bezogen - axialen Abstand zu der Fingerauflage ausgerichtet ist, der so gewählt ist, dass bei einer Positionierung der Dosiervorrichtung mit unterhalb der Unterlippe des Bedieners angeordneter Fingerauflage die Applikationsöffnung in Richtung und auf Höhe eines geöffneten Mundes des Bedieners ausgerichtet ist.

12. Dosiervorrichtung nach dem Oberbegriff des Anspruchs 1 oder nach Anspruch 1, **dadurch gekennzeichnet, dass** der Applikator einen Kolbenfortsatz (6, 6a) aufweist, der in einer unbelasteten Ruhestellung des Applikators radial geführt zumindest teilweise axial in einen offenen Zylinderabschnitt eines Mediumspeichers (3, 3a) hineinragt.
